# EUROPÄISCHE PATENTSCHRIFT

(11) **EP 1 017 689 B1**
(45) Veröffentlichungstag und Bekanntmachung des Hinweises auf die Patenterteilung: **12.03.2003**
(21) Anmeldenummer: 98951436.9
(22) Anmeldetag: 18.09.1998
(51) Int. Cl.: C07D 307/88

(54) **VERFAHREN ZUR REINIGUNG VON PHTHALIDEN**
METHOD FOR PURIFYING PHTHALIDES
PROCEDE DE PURIFICATION DE PHTALIDES

(30) Priorität: 19.09.1997 DE 19741423; 15.10.1997 DE 19745579
(43) Veröffentlichungstag der Anmeldung: 12.07.2000
(73) Patentinhaber: BASF AKTIENGESELLSCHAFT, 67056 Ludwigshafen (DE)
(72) Erfinder: BAUMANN, Dieter, D-69190 Walldorf (DE); PÜTTER, Hermann, D-67433 Neustadt (DE); HANNEBAUM, Heinz, D-67069 Ludwigshafen (DE)
(74) Vertreter: Isenbruck, Günter, Dr.
(86) Internationale Anmeldenummer: EP9805983
(87) Internationale Veröffentlichungsnummer: WO99015515

(56) Entgegenhaltungen:
- WO-A-97/43464
- DE-A- 2 144 419
- DE-A- 2 510 920

## Beschreibung

Die Erfindung betrifft Verfahren zur Herstellung von Phthaliden aus bei der Phthalidherstellung erhaltenen Reaktionsgemischen.

Phthalide, d.h. substituierte Lactone der 2-(Hydroxymethyl)benzoesäure, und Phthalid (Isobenzofuran-1(3H)-on) werden insbesondere als Zwischenprodukte für die Herstellung von Pflanzenschutzmitteln oder Arzneimitteln benötigt.

Verschiedene Verfahren zur Herstellung von Phthaliden sind bekannt. Überwiegend erfolgt die Herstellung von Phthaliden durch elektrochemische Verfahren, durch homogen oder heterogen katalysierte Hydrierung.

In der DE-A 21 44 419 ist ein Verfahren zur elektrochemischen Herstellung von Phthalid beschrieben. Dabei wird eine wäßrige Lösung von Ammoniumphthalamat bei Elektrolysetemperaturen bis 65°C kathodisch an Metallen mit einer Wasserstoffüberspannung größer als der des Kupfers reduziert. Insbesondere wird Reinstblei als Kathodenmaterial eingesetzt. Zur Aufarbeitung werden gegebenenfalls vorhandenes überschüssiges Ammoniak und Lösungsmittel und ein Teil des vorhandenen Wassers abdestilliert. Die zurückbleibende Lösung des primären Reaktionsproduktes, des Ammoniumsalzes der o-Aminomethylbenzoesäure, wird mit einer starken Säure behandelt, wobei Phthalid ausfällt. Durch Filtration wird das Phthalid isoliert. Das noch in Lösung gebliebene Produkt kann mit Benzol extrahiert werden. Das erhaltene Produkt kann durch Umkristallisation aus heißem Wasser weiter gereinigt werden.

In der DE-A 25 10 920 ist ein Verfahren zur elektrochemischen Herstellung von Phthalid beschrieben. Dabei wird eine ammoniakalische wäßrige Lösung von Phthalsäureanhydrid oder Phthalsäure bei Temperaturen bis 100°C an Metallen mit einer Wasserstoffüberspannung größer als der des Kupfers kathodisch reduziert. Das Reaktionsgemisch wird aufgearbeitet, indem man zur Abscheidung des Phthalids aus dem Elektrolysegemisch gegebenenfalls vorhandenes überschüssiges Ammoniak und/oder Wasser abdestilliert und den Rückstand ansäuert, wobei man beim Ansäuern eine Temperatur von 35 bis 100°C einhält.

In der prioritätsälteren, nicht vorveröffentlichten DE-A 196 18 854 ist ein Verfahren zur Herstellung von Phthaliden durch kathodische Reduktion von Phthalsäurederivaten beschrieben. Die Reduktion wird dabei in einer ungeteilten Elektrolysezelle in einem organischem Lösungsmittel, das weniger als 50 Gew.-% Wasser enthält, durchgeführt. Es wird beschrieben, daß die Aufarbeitung durch Destillation, Fällung oder Umkristallisation erfolgen kann. Zudem können die Phthalide in ammoniakalischen wäßrigen Lösungen gelöst werden, wonach die wäßrige Phase abgetrennt und das Phthalid durch Ansäuern aus der wäßrigen Phase wieder ausgefällt wird. In den Beispielen erfolgt die Aufarbeitung durch Abdestillieren des Lösungsmittelgemisches und Vakuumdestillation des Phthalids.

Bei der Destillation muß auf einem hohen Temperaturniveau im Bereich des Siedepunktes der Phthalide gearbeitet werden, wobei das Produkt für einen längeren Zeitraum einer großen thermischen Belastung unterliegt. Zudem ist die Destillation nicht in allen Fällen geeignet, um ein reines Phthalid zu erhalten. Speziell bei der elektrochemischen Herstellung von Phthaliden ausgehend von Phthalsäuremethylester bilden nicht umgesetzte Phthalsäuremethylester mit Phthalid ein Azeotrop, dessen Trennung destillativ nicht möglich ist. Soll der Anteil an im Produkt verbleibendem Phthalsäuremethylester durch entsprechende Reaktionsführung niedrig gehalten werden, so sinkt die Phthalidausbeute, und es können andere Nebenprodukte auftreten.

Bei der Umkristallisation werden die Phthalide in einem Lösungsmittel aufgelöst und aus diesem durch Abkühlung auskristallisiert. Es muß somit ein Lösungsmittel bereitgestellt und in aufwendiger Weise wieder vom reinen Phthalid abgetrennt werden. Zum Handhaben des Lösungsmittels in einem geschlossenen System ist eine aufwendige Verfahrenstechnik erforderlich. Nach der Abtrennung des Kristallisats von der Mutterlauge muß diese aufkonzentriert werden, um weiterverarbeitet werden zu können. Das erhaltene Produkt kann Reste des Lösungsmittels enthalten, das durch Trocknen entfernt werden muß.

Aufgabe der vorliegenden Erfindung ist die Bereitstellung eines Verfahrens zur Herstellung von Phthaliden aus bei der Phthalidherstellung. anfallenden Reaktionsgemischen, das die Nachteile der bekannten Aufarbeitungsverfahren vermeidet. Das Verfahren soll insbesondere ohne den Einsatz von Lösungsmitteln und anderen Hilfsstoffen durchführbar sein, eine geringe thermische Belastung der Phthalide sicherstellen und unter wirtschafltichem Energieaufwand durchführbar sein.

Die Aufgabe wird erfindungsgemäß gelöst durch ein Verfahren zur Herstellung von Phthaliden aus bei der Phthalidherstellung durch kontinuierliche elektrolytische Reduktion erhaltenen Reaktionsgemischen durch
(a) Abdestillieren von Verbindungen mit einem Siedepunkt unterhalb des Siedepunktes des Phthalids aus dem Reaktionsgemisch, wenn diese Verbindungen im Reaktionsgemisch vorliegen, um als Sumpf ein Rohphthalid zu erhalten,
(b) Kristallisation von Phthalid aus einer Schmelze des Rohphthalids.

Die Kristallisation von Phthalid erfolgt aus einer Schmelze des Rohphthalids, das heißt ohne Zuhilfenahme von weiteren Lösungsmitteln und anderen Hilfsstoffen, wie es beispielsweise bei der Umkristallisation notwendig ist.

Das lösungsmittelfreie Rohphthalid wird dabei aus dem Reaktionsgemisch durch Abdestillieren von Verbindungen erhalten, die niedriger als die gewünschten Phthalide sieden. Wenn in den Reaktionsgemischen derartige Verbindungen vorliegen, so werden somit sie vor der Kristallisation destillativ entfernt. Als Sumpf bleibt dabei ein Rohphthalid zurück, wobei die zur Entfernung der niedrigsiedenden Verbindungen notwendige thermische Belastung deutlich geringer ist als die thermische Belastung, die bei einer zusätzlichen nachfolgenden Destillation des Phthalids notwendig wäre. Das zur Kristallisation eingesetzte Rohphthalid ist somit weitgehend oder vorzugsweise vollständig frei von Lösungsmitteln oder niedriger siedenden Verbindungen.

Gemäß einer Ausführungsform der Erfindung erfolgt die Kristallisation an einer gekühlten Fläche, an der die Kristalle wachsen.

Dabei wird das flüssige Rohphthalid mit einer Kühlfläche in Kontakt gebracht, wobei sich bei der Abkühlung der Kühlfläche Phthalidkristalle an dieser bilden. Nach dem Abschluß der Kristallisationsphase wird die verbleibende Flüssigkeit (Mutterlauge) abgeführt. Die Reinheit der auf der Kühlfläche verbleibenden Phthalidkristalle kann noch erhöht werden, indem durch partielles Abschmelzen höher verunreinigte Anteile der Kristalle verflüssigt und abgeführt werden (Schwitzen). Zudem besteht die Möglichkeit, durch Waschen, beispielsweise mit dem eingesetzten Rohphthalid oder mit flüssigem Phthalid eines höheren Reinheitsgrades, die Reinheit der Kristalle auf der Kühlfläche zu erhöhen. Schließlich werden die gereinigten Kristalle durch Erwärmung verflüssigt, und die so erhaltene Schmelze des reinen Phthalids wird von der Kühlfläche abgeführt.

Die Kühlfläche, an der die Kristallisation ausgeführt wird, unterliegt keiner Beschränkung, sie kann eine beliebige geeignete Form aufweisen. Die Temperatur der Schmelze während der Kristallisation beträgt vorzugsweise -10 bis 75°C, besonders bevorzugt 20 bis 70°C. Der Feststoffgehalt im Kristallisator liegt üblicherweise zwischen 10 und 90 g, vorzugsweise zwischen 30 und 80 g, pro 100 g Rohphthalid als Einsatzstoff.

Die Kristallisation kann kontinuierlich oder diskontinuierlich einstufig oder mehrstufig durchgeführt werden.

Die Kristallisation an der gekühlten Fläche kann als statische Kristallisation oder als dynamische Schichtkristallisation durchgeführt werden. Im ersten Fall ruht die eingesetzte Schmelze des Rohphthalids. Ein derartiges statisches Kristallisationsverfahren wird beispielsweise von BEFS/Prokem (Frankreich) oder von Sulzer Chemtech (Schweiz) angeboten. Bei der dynamischen Schichtkristallisation wird die Schmelze des Rohphthalids durch eine Zwangsströmung bewegt. Ein derartiges Verfahren wird beispielsweise von Sulzer Chemtech (Schweiz) angeboten. Bei beiden Verfahrensvarianten sind die gekühlten Flächen im Kristallisationsapparat angeordnet, so daß die sich bildenden Kristalle im Apparat fixiert sind. Vorzugsweise wird die statische Kristallisation durchgeführt, bei der die Schmelze des Rohphthalids ruht und nur natürliche Konvektion auftritt.

Vorteilhaft bei dieser Verfahrensweise ist, daß das Produkt nur einer sehr geringen thermischen Belastung ausgesetzt ist und daß das erforderliche Temperaturniveau niedrig ist, wodurch der Energiebedarf relativ gering ist. Die Trennung der Kristalle von der Mutterlauge kann ohne zusätzlichen apparativen Aufwand erfolgen.

Die Kristallisation an einer gekühlten Fläche wird vorzugsweise mehrstufig als fraktionierte Kristallisation durchgeführt. Dabei kann die fraktionierte Kristallisation auch bei den anderen geeigneten Kristallisationsverfahren, etwa der Suspensionkristallisation, angewendet werden.

Durch die fraktionierte Kristallisation kann die Reinheit der Phthalide erhöht werden, wenn in einer Reinigungsstufe die gewünschte Endreinheit noch nicht erreicht ist. Durch wiederholte Kristallisation der jeweils entstehenden reinen Fraktionen, die daraufhin verflüssigt werden, kann die gewünschte Endreinheit eingestellt werden.

Üblicherweise werden bei fraktionierter Kristallisation alle Stufen, die Kristallisat erzeugen, das reiner ist als das zugeführte Rohphthalid, Reinigungsstufen und alle anderen Stufen Abtriebsstufen genannt. Zwecksmäßigerweise werden mehrstufige Verfahren hierbei nach dem Gegenstromprinzip betrieben, bei dem nach der Kristallisation in jeder Stufe das Kristallisat von der Mutterlauge abgetrennt wird, und dieses Kristallisat der jeweiligen Stufe mit dem nächsthöheren Reinheitsgrad zugeführt wird, während der Kristallisationsrückstand der jeweiligen Stufe mit dem nächstniedrigeren Reinheitsgrad zugeführt wird.

Vorzugsweise wird die fraktionierte Kristallisation mit 2 bis 10 Stufen, besonders bevorzugt mit 2 bis 4 Stufen, insbesondere mit 3 Stufen durchgeführt.

Bei der Suspensionskristallisation wird die Schmelze des Rohphthalids durch Wärmeabfuhr kristallisiert. Die sich bildenden Kristalle sind dabei dispers in der restlichen Flüssigphase (Mutterlauge) verteilt und bilden zusammen mit ihr eine Suspension. Nach Erreichen eines gewünschten Kristallgehalts, üblicherweise 10 bis 40 Gew.-%, werden die Kristalle von der Mutterlauge getrennt. Nach erfolgter Abtrennung können die Kristalle verflüssigt und in einer weiteren Stufe kristallisiert werden.

Die Wärmeabfuhr kann durch Kühlung in einem Wärmeübertrager, vorzugsweise einem Rohbündel-Wärmeübertrager, erfolgen, durch den die Suspension, vorzugsweise rohrseitig, geführt wird. Wenn mit einer Verkrustung der Kühlfläche zu rechnen ist, können mehrere Wärmeübertrager, vorzugsweise 3 Wärmeübertrager, parallel geschaltet werden. Durch Wechselschaltung wird dabei immer einer der Wärmeübertrager außer Betrieb genommen, und durch Erwärmung werden die anhaftenden Phthalidkristalle abgeschmolzen. Hierdurch ist ein kontinuierlicher Betrieb möglich. Beispielsweise kann beim Betrieb der Wärmedurchgangswiderstand des Wärmeübertragers gemessen werden. Wird ein Zustand erreicht, bei dem der Wärmedurchgangswiderstand durch Kristallschichtbildung einen zu hohen Wert annimmt, erfolgt die Umschaltung auf einen zweiten Wärmeüberträger usw. Neben dem Abschmelzen der anhaftenden Kristalle ist auch eine Entfernung durch Durchspülen des Wärmeübertragers mit einer Feed-Lösung des Phthalids möglich. Dadurch ist gleichzeitig eine Vorkühlung des Feeds möglich.

Die für das Kristallwachstum erforderliche Verweilzeit wird in einem Kristallisator, vorzugsweise einem Zwangsumlaufkristallisator, in dessen Suspensionskreislauf die Wärmeübertrager angeordnet sind, geschaffen.

Zudem kann die Wärmeabfuhr aus der Suspension durch einen Wärmeübertrager mit geschabten Kühlflächen (Kratzkühler) erreicht werden. Dabei wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht oft die Möglichkeit, die Wärme über die Wand eines Rührkessels mit wandgängigem Rührer abzuführen. Eine weitere Möglichkeit besteht darin, die Wärmeabfuhr und die Einstellung der Verweilzeit apparativ zu vereinen. Dies erfolgt beispielsweise in einem Kühlscheibenkristallisator, wie er beispielsweise von der Goudsche Machinefabrik B.V. (Niederlande) angeboten wird. Die Wärmeabfuhr erfolgt über gekühlte Platten, die zur Vermeidung von Verkrustungen beidseitig gewischt werden. Die Kühlplatten sind so in einem quaderförmigen Behälter angeordnet, daß sie diesen in äquidimensionale Segmente unterteilen, von denen jedes an zwei Seiten von einer Kühlfläche begrenzt ist. Die Suspension bewegt sich von Segment zu Segment und hat ein sehr enges Verweilzeitspektrum. Das Kühlmedium, das innerhalb der Kühlplatten fließt, wird entgegen der Fließrichtung der Suspension geführt. Dadurch erfährt die Suspension auf ihrem Weg durch den Apparat eine nahezu stetige Temperaturerniedrigung und weist damit einhergehend einen zunehmenden Kristallgehalt auf.

Zur Abtrennung der Mutterlauge von dem auskristallisierten Phthalid eignen sich alle bekannten Verfahren der Fest-Flüssig-Trennung. Beispielsweise können die Kristalle aus der Suspension über eine Zentrifuge, insbesondere eine Schubzentrifuge, oder ein Filter, besonders bevorzugt ein Bandfilter oder ein Drehtellerfilter getrennt werden. Dem Filtrieren oder Zentrifugieren kann eine Voreindickung der Suspension, beispielsweise durch Hydrozyklone, vorgeschaltet werden. Neben ein- oder mehrstufigen Schubzentrifugen sind auch Schneckenzentrifugen oder Schneckenaustragszentrifugen (Dekanter) geeignet. Die Filtration kann diskontinuierlich oder kontinuierlich unter Druck oder bei vermindertem Druck erfolgen. Bei Verwendung von Filternutschen können diese ein Rührwerk aufweisen.

Während und/oder nach der Fest-Flüssig-Trennung können weitere Verfahrensschritte zur Steigerung der Reinheit der Kristalle beziehungsweise des Kristallkuchens vorgesehen werden. Die in Stufe (b) erhaltenen Kristalle können beispielsweise durch Waschen/oder Schwitzen weiter gereinigt werden. Beim Waschen liegt die Waschflüssigkeitsmenge vorzugsweise zwischen 10 und 500 g Waschflüssigkeit/100 g Kristallisat, besonders bevorzugt zwischen 30 und 200 g Waschflüssigkeit/100 g Kristallisat. Geeignete Waschflüssigkeiten sind beispielsweise das flüssige Reinprodukt, das durch Schmelzen der erhaltenen Kristalle erhalten wird, oder das flüssige Rohphthalid. Das Waschmittel sollte eine höhere Reinheit als die Mutterlauge, aus der das Kristallisat abgetrennt wurde, aufweisen. Das Durchführen des Waschens oder Schwitzens kann unter Umständen eine weitere Reinigungs- beziehungsweise Kristallisationsstufe einsparen.

Das Waschen kann in hierfür üblichen Apparaten erfolgen. Vorteilhafterweise werden Waschkolonnen, in denen die Abtrennung der Mutterlauge und das Waschen in einem Apparat erfolgen, Zentrifugen, die ein- oder mehrstufig betrieben werden können, oder Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Beim Schwitzen handelt es sich um ein lokales Abschmelzen verunreinigter Bereiche der Kristalle. Vorteilhafterweise beträgt die Schwitzmenge zwischen 5 und 35 g abgeschmolzenes Kristallisat/100 g Kristallisat vor dem Schwitzen. Besonders bevorzugt ist die Durchführung des Schwitzens auf Bandfiltern. Auch die Durchführung einer Kombination aus Waschen und Schwitzen in einem Apparat kann geeignet sein.

Eine weitere Möglichkeit zur Erhöhung der Reinheit besteht darin, das Kristallisat nach der Abtrennung anzumaischen und erneut einer Trennung zu unterziehen. Das Anmaischen kann im Reinprodukt oder in der Schmelze des Rohphthalids erfolgen.

Die Reinheit des erhaltenen Phthalids beträgt vorzugsweise 97 bis 99,9 Gew.-%, insbesondere 98,5 bis 99,5 Gew.-%.

Besonders bevorzugt wird die Kristallisation des Phthalids aus der Schmelze des Rohphthalids in Stufe (b) durch dreistufige statische Schmelzekristallisation durchgeführt.

Das Reaktionsgemisch, aus dem die Phthalide isoliert werden, stammt aus der kontinuierlichen elektrolytischen Reduktion, wie sie beispielsweise in DB-A 21 44 419 und DE-A 25 10 920 oder DE-A 196 18 854 beschrieben ist. Das Reaktionsgemisch kann dabei Lösungsmittel, Leitsalze, anodische Depolarisatoren, Mediatoren oder Gemische davon enthalten.

Bei der elektrolytischen Reduktion werden insbesondere Phthalsäure oder Phthalsäurederivate, bei denen die Carboxygruppen durch Einheiten ersetzt sein können, die von Carboxygruppen in einer Kondensationsreaktion ableitbar sind, und eines oder mehrere der Wasserstoffatome der o-Phenylen-Einheit der Phthalsäure durch inerte Reste substituiert sein können, in einem Elektrolyten gelöst an einer Kathode in einer ungeteilten Elektrolysezelle reduziert.

Als Ausgangsverbindungen für die Herstellung der Phthalide werden insbesondere solche der allgemeinen Formel (I) 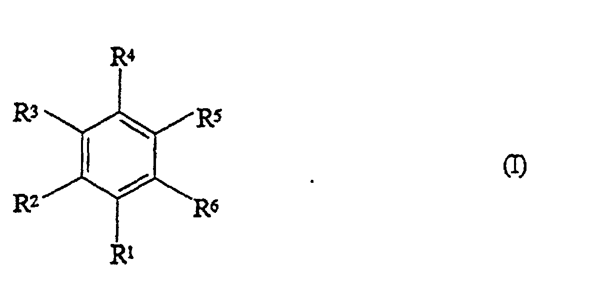 eingesetzt, in der die Substituenten die folgende Bedeutung haben:
- R¹, R², R³, und R⁴:: unabhängig voneinander Wasserstoff, C₁- bis C₄-Alkyl oder Halogen
- R⁵, R⁶:: (a) unabhängig voneinander -COOH oder COOX, wobei X für C₁- bis C₄-Alkyl steht,
(b) einer der Substituenten R⁵ oder R⁶ -COONY₂ und der andere Substituent CONH₂, wobei Y für C₁- bis C₄-Alkyl oder Wasserstoff steht,
(c) R⁵ und R⁶ zusammen -CO-O-CO-.

Besonders bevorzugt sind die Derivate der Phthalsäure, bei denen R¹, R², R³ und R⁴ Wasserstoff bedeuten und darunter insbesondere die Phthalsäuredi-(C₁- bis C₃-alkyl)-ester, vor allem der Phthalsäuredimethylester.

Die elektrochemische Umsetzung dieser Ausgangsprodukte kann beispielsweise nach der Methode erfolgen, wie sie in der DE-A 196 18 854 beschrieben ist.

Bei diesem Verfahren werden als Elektrodenmaterialien (sowohl Kathode als auch Anode) vor allem handelsübliche Elektroden aus Graphit oder Kohle eingesetzt.

Bei dem Elektrolyten handelt es sich üblicherweise um eine 2 bis 40 Gew.-%ige Lösung der Phthalsäure oder eines Phthalsäurederivates in einem organischen Lösungsmittel oder einer Mischung aus einem organischen Lösungsmittel und Wasser, wobei die Mischung im allgemeinen weniger als 50 Gew.-%, bevorzugt weniger als 25 Gew.-%, besonders bevorzugt weniger als 5 Gew.-% Wasser enthält.

Als organische Lösungsmittel eignen sich insbesondere aliphatische C₁- bis C₄-Alkohole, insbesondere Methanol oder Ethanol oder Mischungen derartiger Alkohole mit einem Carbonsäureamid wie Dimethylformamid oder tert.-Butylformamid.

Als Leitsalze enthalten die Elektrolyte beispielsweise quarternäre Ammoniumsalze, wie Tetra-(C₁- bis C₄-alkyl)ammoniumhalogenide oder -tetrafluoroborate und bevorzugt Methyltributylammonium- oder Methyltriethylammoniummethylsulfat, üblicherweise in Mengen von 0,4 bis 10 Gew.-%, bezogen auf den Elektrolyt.

Für den anodischen Koppelprozeß empfiehlt es sich, als anodischen Depolarisator übliche organische Verbindungen einzusetzen, deren Eignung für die elektrochemische Oxidation dem Fachmann allgemein bekannt ist. Einige der anodischen Koppelprozesse werden bevorzugt in Anwesenheit eines Mediators durchgeführt. Geeignete anodische Koppelprozesse werden beispielsweise in D. Kyriakou, Modem Electroorganic Chemistry, Springer, Berlin 1994 in Kapitel 4.2 beschrieben.

Als anodische Koppelprozesse eignen sich insbesondere die Oxidationen von C-O- oder C-N-Einfach- oder -Doppelbindungen, z.B. die Oxidation von Carbonsäuren, oder die oxidative C-C-Verknüpfung insbesondere von Naphthalinen oder aktivierten CH-Gruppen sowie die Oxidation von an einen aromatischen Kern gebundenen Methylgruppen zu Aldehyden.

Als besonders günstig hat sich der Einsatz von Methylbenzol oder kernsubstituierten Derivaten des Methylbenzols erwiesen, bei dem 1 bis 3 Wasserstoffatome des Phenylrestes durch C₁- bis C₆-Alkylreste oder C₁- bis C₄-Alkoxyreste ersetzt sein können. Beispiele für derartige anodische Depolarisatoren sind p-Xylol und p-tert-Butyltoluol.

Bei der Herstellung von Aldehyden als Koppelprodukten empfiehlt sich der Einsatz der genannten Alkohole als Lösungsmittel, da die Aldehyde acetalisiert und vor einer Weiteroxidation geschützt werden.

Als Mediatoren eignen sich insbesondere Halogenverbindungen, vor allem Bromide oder Iodide.

Was die sonstigen Verfahrensparameter wie Temperatur und Stromdichte betrifft, so sind diese unkritisch, solange sie sich im für elektrochemische Umsetzungen organischer Verbindungen üblichen Rahmen bewegen. Sie sind beispielsweise in der DE-A 25 10 920 näher spezifiziert.

Wenn der Umsatz so weit fortgeschritten ist, daß das Molverhältnis (M), gebildet aus dem Anteil an Phthalid und der Summe aus dem Anteil an Phthalid und der Phthalidsäure oder den Phthalsäurederivaten im Elektrolyten 0,8:1 bis 0,99:1 bevorzugt 0,88:1 bis 0,95:1 beträgt, trägt man den Elektrolyten aus der Elektrolysezelle aus.

Bei der kontinuierlichen Durchführung des Verfahrens wird man zweckmäßigerweise den kontinuierlichen Austrag des Elektrolyten und die kontinuierliche Ergänzung der inerten Bestandteile des Elektrolyten, wie der Lösungsmittel und Leitsalze sowie der Ausgangsprodukte für die elektrochemische Reaktion, so aufeinander und auf die Reaktionsgeschwindigkeit abstimmen, daß die Konzentration aller Bestandteile des Elektrolyten weitgehend konstant bleibt. Dies gilt insbesondere für das Molverhältnis (M), das sich innerhalb des definitionsgemäßen Bereiches bewegt.

Im allgemeinen wird der ausgetragene Elektrolyt, d.h. das Reaktionsgemisch vor der Kristallisation destillativ aufgearbeitet, wie es vorstehend beschrieben ist.

Die beim erfindungsgemäßen Reinigungsverfahren entstehende Mutterlauge und die gegebenenfalls erhaltene Waschlösung können ohne weitere Aufarbeitung in die Elektrolysezelle zurückgeführt werden, da sie im wesentlichen aus einem Gemisch von Phthalid und der entsprechenden Ausgangsverbindung bestehen.

## Patentansprüche

1. Verfahren zur Herstellung von Phthaliden aus bei der Phthalidherstellung durch kontinuierliche elektrolytische Reduktion anfallenden Reaktionsgemischen durch
(a) Abdestillieren von Verbindungen mit einem Siedepunkt unterhalb des Siedepunktes des Phthalids aus dem Reaktionsgemisch, wenn diese Verbindungen im Reaktionsgemisch vorliegen, um als Sumpf ein Rohphthalid zu erhalten,
(b) Kristallisation von Phthalid aus einer Schmelze des Rohphthalids.

2. Verfahren nach Anspruch 1, **dadurch gekennzeichnet, daß** die Kristallisation an einer gekühlten Fläche durchgeführt wird, an der die Kristalle wachsen.

3. Verfahren nach Anspruch 1, **dadurch gekennzeichnet, daß** die Kristallisation als Suspensionskristallisation durchgeführt wird.

4. Verfahren nach einem der Ansprüche 1 bis 3, **dadurch gekennzeichnet, daß** die Kristallisation mehrstufig als fraktionierte Kristallisation durchgeführt wird.

5. Verfahren nach einem der Ansprüche 1 bis 4, **dadurch gekennzeichnet, daß** die in Stufe (b) erhaltenen Kristalle durch Waschen und/oder Schwitzen weiter gereinigt werden.

6. Verfahren nach einem der Ansprüche 1 bis 5, **dadurch gekennzeichnet, daß** das Reaktionsgemisch Lösungsmittel, Leitsalze, anodische Depolarisatoren, Mediatoren oder Gemische davon enthält.

## Claims

1. The process for recovering a phthalide from an as-obtained continuous electrolytic reduction phthalide synthesis reaction mixture by
(a) distilling compounds having a boiling point below the boiling point of said phthalide from said reaction mixture, provided such compounds are present in said reaction mixture, to obtain a crude phthalide as bottom product,
(b) crystallizing said phthalide from a melt of said crude phthalide.

2. The process of claim 1, wherein said crystallizing is effected on a cooled surface on which the crystals grow.

3. The process of claim 1, wherein said crystallizing is effected as a suspension crystallization.

4. The process of any of claims 1 to 3, wherein said crystallizing is effected in multiple stages as a fractional crystallization.

5. The process of any of claims 1 to 4, wherein the crystals obtained in step (b) are further purified by washing and/or sweating.

6. The process of any of claims 1 to 5, wherein said reaction mixture comprises solvents, conducting salts, anodic depolarizers, mediators or mixtures thereof.

## Revendications

1. Procédé de préparation de phtalides à partir de mélanges réactionnels obtenus au cours de la préparation de phtalides par réduction électrolytique continue, par
(a) séparation par distillation de composés ayant un point d'ébullition en dessous du point d'ébullition du phtalide à partir du mélange réactionnel, lorsque ces composés sont présents dans le mélange réactionnel, pour obtenir comme produit de fond de colonne un phtalide brut,
(b) cristallisation du phtalide dans une masse fondue du phtalide brut.

2. Procédé suivant la revendication 1, **caractérisé en ce que** la cristallisation est effectuée sur une surface refroidie sur laquelle les cristaux croissent.

3. Procédé suivant la revendication 1, **caractérisé en ce que** la cristallisation est effectuée sous la forme d'une cristallisation en suspension.

4. Procédé suivant l'une des revendications 1 à 3, **caractérisé en ce que** la cristallisation est effectuée en plusieurs étapes, sous la forme d'une cristallisation fractionnée.

5. Procédé suivant l'une des revendications 1 à 4, **caractérisé en ce que** les cristaux obtenus dans l'étape (b) sont davantage purifiés par lavage et/ou exsudation.

6. Procédé suivant l'une des revendications 1 à 5, **caractérisé en ce que** le mélange réactionnel contient du solvant, des sels conducteurs, des agents de dépolarisation anodiques, des médiateurs ou leurs mélanges.
